# EUROPEAN PATENT SPECIFICATION

(11) **EP 1 981 460 B1**
(45) Date of publication and mention of the grant of the patent: **15.09.2010**
(21) Application number: 07705843.6
(22) Date of filing: 09.02.2007
(51) Int. Cl.: A61F 13/42

(54) **ABSORBENT ARTICLE WITH SENSATION MEMBER**
SAUGFÄHIGER ARTIKEL MIT WAHRNEHMUNGSELEMENT
ARTICLE ABSORBANT MUNI D'UN ELEMENT DE SENSATION

(30) Priority: 10.02.2006 US 351745
(43) Date of publication of application: 22.10.2008
(73) Proprietor: The Procter and Gamble Company, Cincinnati, Ohio 45202 (US)
(72) Inventor: ROE, Donald Carroll, West Chester, Ohio 45069 (US); NISHIKAWA, Masaharu, Cincinnati, Ohio 45249 (US); NANDREA, Jennifer Joan, Cincinnati, Ohio 45218 (US); LIU, Kuang-Kai, Cincinnati, Ohio 45249 (US)
(74) Representative: Heide, Ute
(86) International application number: PCT/IB2007/050440
(87) International publication number: WO 2007/091225

(56) References cited:
- EP-A- 1 287 799
- EP-A2- 0 937 446
- WO-A-02/091968
- WO-A-03/045298
- WO-A-2005/041834
- WO-A-2005/102239

## Description

### FIELD OF THE INVENTION

The present disclosure generally reflates to absorbent articles, including diapers, training pants, pull-on diapers, absorbent inserts, diaper holders and lingers, and the like, and in particular to an absorbent article with a sensation member, which may be adapted for use in urinary toilet training.

### BACKGROUND OF THE INVENTION

Absorbent articles are well known in the art. These articles typically have an absorbent assembly held or positioned in proximity to the body of a wearer during use in order to capture and absorb bodily exudates discharged from the wearer. Typical absorbent articles include a topsheet facing the wearer, which permits fluid exudates to pass through, and a backsheet, which prevents the exudates from escaping from the absorbent article.

Disposable absorbent articles such as diapers are designed to absorb and contain bodily waste in order to present soiling of the body and clothing of the wearer. Disposable diapers typically comprise a single design available in different sizes to fit a variety of wearers ranging from newborns to toddlers undergoing toilet training. The design of the diaper typically affects performance, such as the ability to absorb and contain bodily waste. The fit of the diaper on the wearer's body is typically affected by, for example, the size of the diaper waist opening, the size of the openings around the thighs, and the length or "pitch" of the diaper.

The toilet braining stage may be referred to as the "point of exit" from the diaper product category because toddlers who have successfully completed toilet training typically no longer wear diapers. The age at which children are toilet trained in "developed" countries has increased steadily over the past several decades and is now in the range of about 24-48 months. One reason for which toilet training has become delayed is that significant technical improvements have been made in diaper dryness and comfort. For example, when wearing a typical modem diaper, the child may have dry skin even after one or more occurrences of urination. As a result, the child may feel little or no discomfort and often may not even be aware that he or she has urinated.

Exemplary absorbent articles comprising wetness sensation members for use in urinary toilet training are described in WO 2005/041834 and in WO 03/045298.

WO 02/091968 describes wearable articles having a temperature change element providing a cool / wet signal when wetted which causes discomfort to the wearer.

EP 0 937 446 relates to training pants for infants which include a wetness retaining sheet substantially made of hydrophilic fibers and are adapted to be washed or reused.

Some parents may have the child wear cotton training patent or cotton underwear during urinary training so the child feels discomfort following urination in his or her "pants." It is believed that such discomfort assists with learning or provides motivation to learn to voluntarily retain urine. Cloth training pants leave the skin wet and, due to their high breathability, promote evaporative cooling of the skin, further enhancing discomfort. The current tradeoff in this approach, however, if that cloth training pants have poor urine containment, often leading to wet clothing and wet surroundings, e.g., carpeting, furniture, etc. Clearly, there is a need to provide a training signal to the child undergoing urinary toilet training while preventing urine leakage and unnecessary changes of clothing.

Thus, it would be desirable to provide an article that can facilitate urinary toilet training by enhancing a wearer's awareness that urination has occurred while at the same time providing the protection of an absorbent article to prevent soiling of the wearer's clothing and surroundings. Particularly, it would be desirable to provide such an article in a form that also provides an effective signal of urination by ensuring that the wearer feels an uncomfortable sensation resulting from urination.

### SUMMARY OF THE INVENTION

In one aspect, an absorbent article has a waist region and a crotch region. The article includes a backsheet having a longitudinal axis, a topsheet attached to the backsheet and having a body-facing surface, and an absorbent core disposed between the backsheet and the topsheet. The article also includes a sensation member with first and second joining regions spaced from each other along the longitudinal axis, the first joining region being attached to the topsheet at the waist region and the second joining region being attached to the topsheet at the crotch region. The sensation member consists essentially of a layer of hydrophilic material.

In another aspect, an absorbent article has a waist region and a crotch region. The article includes a backsheet having a longitudinal axis, a topsheet attached to the backsheet and having a body-facing surface, and an absorbent core disposed between the backsheet and the topsheet. The article also includes a sensation member with first and second joining regions spaced from each other along the longitudinal axis, the first joining region being attached to the topsheet at the waist region and the second joining region being attached to the topsheet at the crotch region. The sensation member includes a support layer having a body-facing surface and an opposite surface facing the topsheet, and an active component on one of the body-facing surface or the opposite surface of the sensation member.

In yet another aspect, an absorbent article includes a backsheet having a longitudinal axis, a topsheet attached to the backsheet and having a body-facing surface and an opposite surface facing the backsheet, and an absorbent core disposed between the backsheet and the topsheet. The article also includes a sensation member comprising an active component disposed on a region of the topsheet on one of the body-facing surface and the opposite surface, the region of the topsheet spaced from the absorbent core.

In a further aspect, an absorbent article includes a backsheet having a longitudinal axis, a topsheet attached to the backsheet and having a body-facing surface, an absorbent core disposed between the backsheet and the topsheet, and first and second spaced barrier leg cuffs attached to the topsheet parallel to the longitudinal axis. The article also includes a sensation member having first and second spaced sides, the first side being attached to the first barrier leg cuff and the second side being attached to the second barrier leg cuff with the sensation member spaced a distance from the body-facing surface of the topsheet.

Additional aspects of the disclosure are defined by the claims of this patent.

### BRIEF DESCRIPTION OF THE DRAWINGS

While the specification concludes with claims particularly pointing out and distinctly claiming the subject matter that is regarded as the present invention, it is believed that the invention will be more fully understood from the following description taken in conjunction with the accompanying drawings. In the accompanying drawing figures, like reference numerals identify like elements, which may or may not be identical in the several exemplary embodiments that are depicted. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings are necessarily to scale.
Fig. 1 is a plan view of an absorbent article with a section of a topsheet removed to expose an underlying absorbent core;
Fig. 2 is a perspective view of an exemplary absorbent article shown in its relaxed, contracted state, *i*.*e*., with the contraction induced by elastic members;
Fig. 3a is a plan view of an absorbent article having a sensation member according to an embodiment of the present disclosure;
Fig. 3b is a cross-sectional view of the article shown in Fig. 3a illustrating the layers of the sensation member;
Fig. 4a is an isometric view of the article shown in Fig. 3a illustrating a first exemplary attachment of the sensation member;
Fig. 4b is an isometric view of the article shown in Fig. 3a illustrating a second exemplary attachment of the sensation member;
Fig. 5a is a plan view of an absorbent article having a sensation member according to another embodiment of the present disclosure;
Fig. 5b is a cross-sectional view of the article shown in Fig. 5a illustrating the layers of the sensation member;
Fig. 6a is a plan view of an absorbent article having a sensation member according to a further embodiment of the present disclosure;
Fig. 6b is a cross-sectional view of the article shown in Fig. 6a illustrating the layers of the sensation member.

### DETAILED DESCRIPTION OF THE INVENTION

### Definitions

As used herein, the following terms have the following meanings:
The term "absorbent article" refers to a device that absorbs and contains liquid, and more specifically, refers to a device that is placed against or in proximity to the body of the wearer to absorb and contain the various exudates discharged from the body.
The term "associated with", in relation to highlighting, refers at least to highlighting that is on an element or to highlighting that is disposed proximate to an element.
The term "associative correlation" refers to establishing a mutual or reciprocal relation between the visible highlighting and that with which it is being associatively correlated so that an association, i.e. a mental connection or bond, is formed between the two. This term is used in the context of associatively correlating the respective visible forms of the visible highlighting and an externally visible graphics in or on the absorbent article as well as in the context of associatively correlating the visible highlighting or graphics with the concept of urinary toilet training, For example, associatively correlated graphics may serve in concert to draw attention to an opportunity for urinary toilet training when an absorbent article is viewed prior to its being worn, to provide an externally visible reminder of the presence of the sensation member in the interior of the absorbent article while it is being worn, etc. Similarly, visible highlighting that provides a visual reference to a topic related to urinary toilet training, such as dryness, wetness, or protection from wetness, may serve to associatively correlate the visible highlighting to the concept of urinary toilet training and thereby facilitate an opportunity for urinary toilet training.
The term "attached" refers to elements being connected or united by fastening, adhering, bonding, etc. by any method suitable for the elements being attached together and their constituent materials. Many suitable methods for attaching elements together are well-known, including adhesive bonding, pressure bonding, thermal bonding, mechanical fastening, etc. Such attachment methods may be used to attach elements together over a particular area either continuously or intermittently.
The term "caregiver" refers to a person other than the child, such as, a parent, babysitter, family member, teacher, day care worker, or other person who is able to provide sufficient assistance to the child to complete a personal hygiene task.
The term "character image" refers to a graphic containing an anthropomorphic image, and in particular an image having or suggesting human form or appearance which ascribes human motivations, characteristics or behavior to inanimate objects, animals, natural phenomena, toys, cartoon characters, or the like. The character image may be associated with popular characters in the media, advertising or well known in a particular culture. Ideally they are characters that the user, particularly if a child, cares about and wants to identify with.
The term "coloration" refers to the arrangement or degree of coloring especially when used to visibly differentiate an object or a portion of an object in order to visibly highlight it.
The term "coloring" refers to the effect produced by applying or combining colors in and/or on an object or a portion of an object.
The term "diaper" refers to an absorbent article generally worn by infants and incontinent persons about the lower torso and having the general form of a sheet, different portions of which are fastened together to encircle the waist and the legs of the wearer.
The term "disposable" refers to absorbent articles that generally are not intended to be laundered or otherwise restored or reused as absorbent articles, i.e., they are intended to be discarded after a single use and, preferably, to be recycled, composted or otherwise disposed of in an environmentally compatible manner.
The term "disposed" is used to mean that an element(s) is formed (joined and positioned) in a particular place or position as a unitary structure with other elements or as a separate element joined to another element.
The term "graphic" refers to a product of graphic art or a graphic representation in a pictorial form. A graphic may be a symbol, shape, image, text, or other form of indicia.
The terms "interactively interrelated", "interactively unrelated", "related in subject matter", "unrelated in subject matter", and "related by a common story line" are intended to have the same meanings as in U.S. Patent No. 6,297,424, 6,635,797, and 6,307,119.
The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside". Also, when the absorbent article is oriented such that its interior faces upward, e.g., when it is laid out in preparation for setting the wearer on top of it, synonyms include "upper" and "lower" and "top" and "bottom", respectively.
The term "lateral" or "transverse" refers to a direction running at a 90 degree angle to the longitudinal direction and includes directions within ±45° of the lateral direction.
The term "longitudinal" refers to a direction running parallel to the maximum linear dimension of the article and includes directions within ±45° of the longitudinal direction.
The terms "pre-literate" and "incapable of reading" are used interchangeably herein to mean the inability of a child to correctly understand, comprehend and follow prompts written in a language that the child can speak without assistance of a caregiver. The ability of a child to recognize letters and/or read one or two isolated words still means that the child is "incapable of reading" since he or she is unable to understand, comprehend and follow such written prompts, without assistance. However, this definition of "incapable of reading" does not exclude the child from being able to understand, comprehend and follow visual prompts which are presented in the form of drawings, icons, symbols, gestures, cartoons and the like.
The term "refastenable" refers to the property of two elements being capable of releasable attachment, separation, and subsequent releasable reattachment without substantial permanent deformation or rupture.
The terms "releasably attached," "releasably engaged," and variations thereof refer to two elements being connected or connectable such that the elements tend to remain connected absent a separation force applied to one or both of the elements, and the elements being capable of separation without substantial permanent deformation or rupture. The required separation force is typically beyond that encountered while wearing the absorbent garment.
The term "solid coloring" refers to the unbroken, i.e., uninterrupted, coloring of an area as contrasted with the discrete line-like form of some graphics.
The term "toilet training" refers to the development of continence, which is the ability to voluntarily retain one's urine and feces. Individuals who are incontinent are unable to voluntarily retain their bodily discharges and, instead, urinate and defecate reflexively. For example, newborn babies are incontinent. Coincident with the development of continence, children typically develop the ability to voluntarily urinate and defecate, and cease reflexive elimination. This development of continence and of voluntary elimination, in place of reflexive elimination, may be accelerated and/or guided by caregivers through associative and conditioning techniques of training the child. For the purpose of the present disclosure, the term "toilet training" is used to denote training both for continence, itself, and for the voluntary elimination that is associated with continence. It is also noted that the term "toilet training" is synonymous with the term "potty training".
The term "training pants" refers to an absorbent article generally worn by infants and incontinent persons about the lower torso and having the general form of a pair of short pants that can be applied or removed from the wearer without unfastening.
The term "unitary" refers to an absorbent article that is formed of separate parts united together to form a coordinated entity so as to not require separate manipulative parts like a separate holder and liner.
The term "visible" refers to the quality of being capable of being seen by the naked eye under conditions of normal room lighting or in natural light during the daytime. Becoming "more visible" or "less visible" means changing in visibility to a noticeable extent when viewed under a generally constant or equal lighting condition.
The term "visible highlighting" refers to the visible differentiation of an object such that it noticeably stands out from its surroundings, e.g., by differing in coloration, hue, or tint, by differing in lightness, darkness, or contrast, by differing due to the presence or absence of graphical or solid color forms, or by any other variation serving to create noticeable visible differentiation.
The terms "water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water to pass through its thickness in the absence of a forcing pressure. Conversely, the term "water-impermeable" refers to a layer or a layered structure through the thickness of which liquid water cannot pass in the absence of a forcing pressure. A layer or a layered structure that is water-impermeable according to this definition may be permeable to water vapor, i.e., may be "water vapor-permeable". Such a water vapor-permeably layer or layered structure is commonly known in the art as "breathable". As is well known in the art, a common method for measuring the permeability to water of the materials typically used in absorbent articles is a hydrostatic pressure test, also called a hydrostatic head test or simply a "hydrohead" test. Suitable well known compendial methods for hydrohead testing are approved by INDA (formerly the International Nonwovens and Disposables Association, now The Association of the Nonwoven Fabrics Industry) and EDANA (European Disposable And Nonwovens Association).
The term "x-y plane" refers to the generally planar structure of a sheet material defined by its length and width and lies between the sheet material's two major surfaces regardless of whether or not the sheet material is flat or curved.
The term "z-direction" refers to the direction through the thickness of a sheet material and generally orthogonal to the x-y plane.

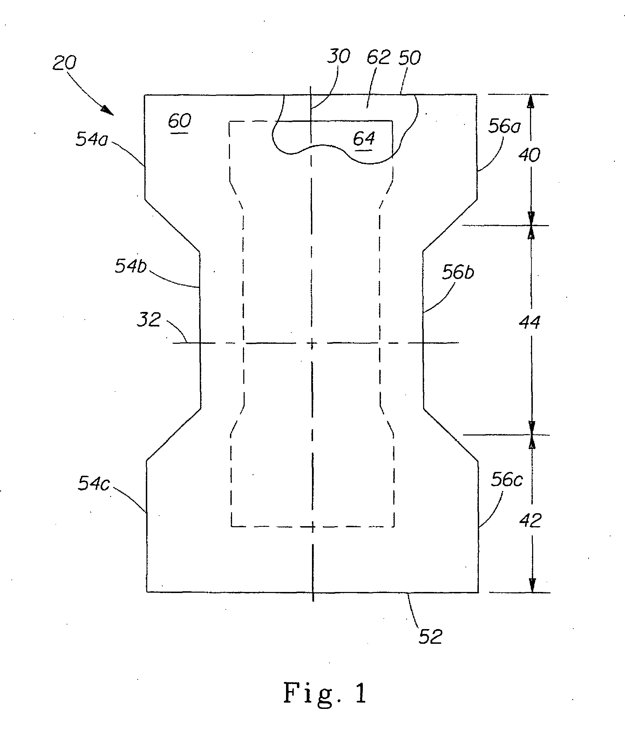

Fig. 1 is a plan view of an exemplary disposable absorbent article 20 in its flat, uncontracted state, i.e., without elastic-induced contraction. Portions of the article 20 have been cut away to more clearly show the underlying structure of the disposable absorbent article 20. As illustrated, the portion of the disposable absorbent article 20 that contacts the wearer faces the viewer (i.e., showing the interior or inner side of the article). The disposable absorbent article 20 has a longitudinal axis 30 and a transverse axis 32.

One end portion of the disposable absorbent article 20 is configured as a first waist region 40 of the disposable absorbent article 20. The opposite end portion is configured as a second waist region 42 of the disposable absorbent article 20. The waist regions 40 and 42 generally comprise those portions of the disposable absorbent article 20 which, when worn, encircle the waist of the wearer. The waist regions 40 and 42 may include elastic elements such that they gather about the waist of the wearer to provide improved fit and containment. An intermediate portion of the disposable absorbent article 20 is configured as a crotch region 44, which extends longitudinally between the first and second waist regions 40 and 42. The crotch region 44 is that portion of the disposable absorbent article 20 which, when the disposable absorbent article 20 is worn, is generally positioned between the legs of the wearer.

The disposable absorbent article 20 has a laterally extending first waist edge 50 in the first waist region 40 and a longitudinally opposing and laterally extending second waist edge 52 in the second waist region 42. The disposable absorbent article 20 has a first side edge 54 and a laterally opposing second side edge 56, both side edges extending longitudinally between the first waist edge 50 and the second waist edge 52. The portion of the first side edge 54 in the first waist region 40 is designated 54a, the portion in the crotch region 44 is designated 54b, and the portion in the second waist region 42 is designated 54c. The corresponding portions of the second side edge 56 are designated 56a, 56b, and 56c, respectively.

The disposable absorbent article 20 preferably comprises a water-permeable topsheet 60, a water-impermeable backsheet 62, and an absorbent assembly or core 64, which may be disposed between the topsheet 60 and the backsheet 62 with the topsheet 60 attached to the backsheet 62. The topsheet 60 may be fully or partially elasticized or may be foreshortened so as to provide a void space between the topsheet 60 and the core 64. As explained below, a fully or partially elasticized topsheet 60 may also to tend to draw a sensation member against the skin of the wearer. Exemplary structures including elasticized or foreshortened topsheets are described in greater detail in U.S. Patent Nos. 4,892,536, 4,990,147, 5,037,416, and 5,269,775, among others.

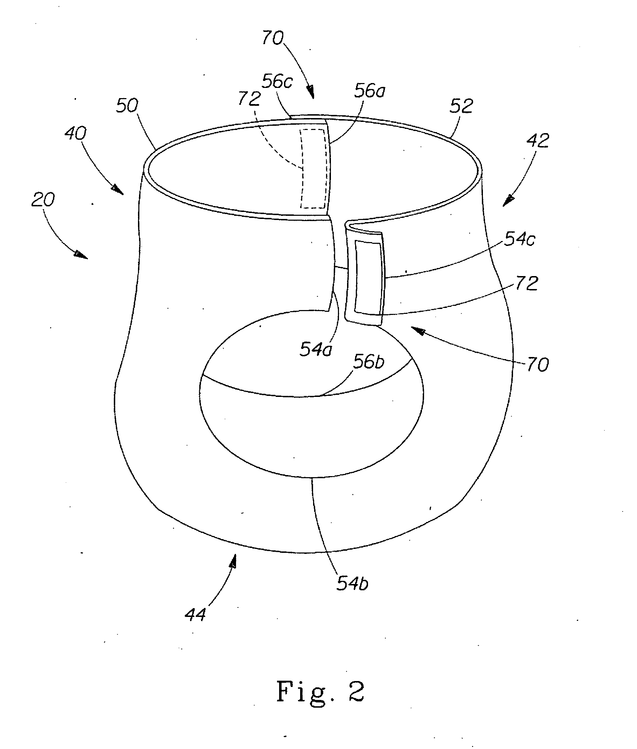

Fig.2 illustrates the article illustrated in Fig. 1 configured to as it would be worn. The disposable absorbent article 20 may be sealed at the sides so as to be configured as illustrated in Fig.2. However, the article 20 may instead include refastenable side seams 70 that can be used to fasten the waist regions 40, 42 together. According to one exemplary embodiment, the waist regions 40, 42 may be fastened at the sides to apply the article like a diaper. According to a further exemplary embodiment, illustrated in Fig. 2, the side seams 70 may include fasteners 72 that can be used to configure the article like a pair of pull-on training pants or disposable pants.

As illustrated, the fasteners 72 may be disposed on the interior of the disposable absorbent article 20 in the second waist region 42 adjacent to the portion 54c of the first side edge 54 and adjacent to the portion 56c of the second side edge 56. The portion 54c of the side edge 54 is shown in an open condition, such as prior to closing and fastening or after being reopened. The portion 56c of the opposing side edge 56 is shown fastened, i.e., forming a pants configuration. In Fig. 2, the second waist region 42 overlaps the first waist region 40 when they are fastened together.

The fasteners 72 may be formed of any material and in any form that will releasably attach to the mating surface of the opposing waist region when pressed against it. For example, the primary fastening component may be a mechanical fastener that releasably engages with the mating surface, such as by means of a plurality of hooks engaging with loops formed by fibers in a nonwoven sheet. Alternatively, the primary fastening component may be an adhesive that releasably adheres to the mating surface.

Still other variations are also possible. For example, the fasteners 72 may be disposed on the interior of the article 20 in the first waist region 40 such that the first waist region 40 overlaps the second waist region 42 when they are fastened together. As another example, the fasteners 70 may be disposed on the exterior of the article 20 rather than on the interior. As a further example, the fasteners 70 may be used with a specific mating fastener surface particularly suited for cooperation with the fasteners 70 (e.g., a loop layer that works with a hook fastener, or a layer particularly treated to provide a suitable contacting surface for a specific adhesive). Additionally exemplary fasteners and fastener arrangements, the fastening components forming these fasteners, and the materials that are suitable for forming fasteners are described in U.S. Published Application Nos. 2003/0060794 and 2005/0222546 and U.S. Patent No. 6,428,526, among others.

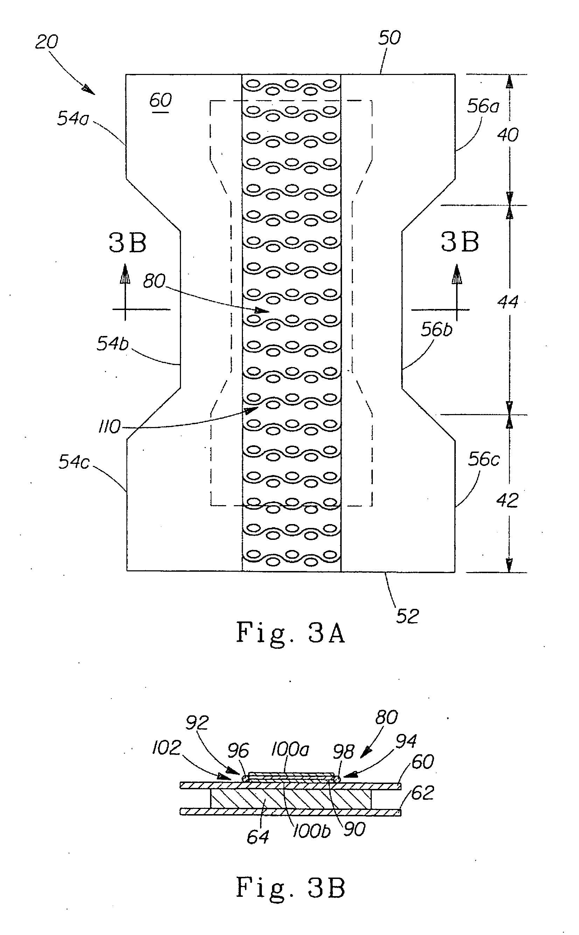

According to the present disclosure, the exemplary article 20, such as is illustrated in Figs. 1 and 2, may be combined with or assembled to include a sensation member 80. Several embodiments, some with one or more variations, are illustrated in Figs. 3a, 3b, 4a, 4b, 5a, 5b, 6a, and 6b. Elements common to all embodiments are numbered similarly in all Figures, while those elements unique to each embodiment are numbered differently, with the sensation member according to a first embodiment being numbered as 80, a second embodiment as 180, and a third embodiment as 280. In addition, while the exemplary absorbent articles illustrated each include a single sensation member, the articles may include a plurality of sensation members according to other embodiments.

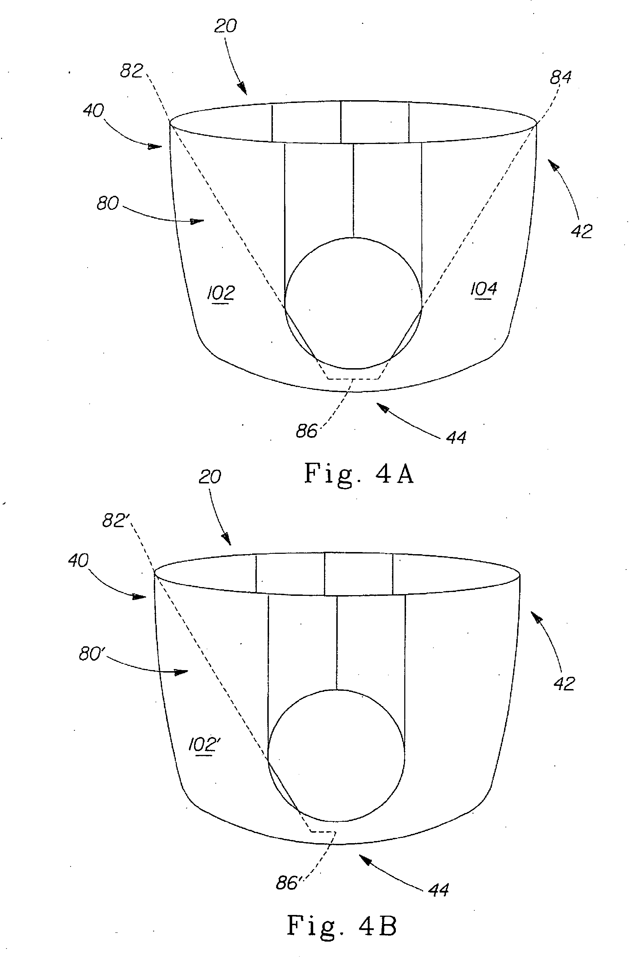

Turning then to the first embodiment of the exemplary sensation member 80, shown in Figs. 3a, 3b, and 4a, the sensation member 80 illustrated is a structure that is formed separately from, but discretely attached to, the topsheet 60. In particular, and as seen best in Fig. 4a, the sensation member 80 has a first laterally extending joining region or end 82 attached to the first waist region 40, and a second longitudinally opposing and laterally extending joining region or end 84 attached to the second waist region 42. In addition, the sensation member 80 may have a center joining region 86 that may be attached to the crotch region 44. It is believed that the attachment of the member 80 to the crotch region 44 may assist in stabilizing the member 80, in facilitating fitting of the article 20 to the wearer, in preventing interference with bowel movements and in ensuring good contact with the wearer's skin.

As seen in Fig. 3b, the sensation member 80 may comprise a hydrophilic layer 90, alone or in combination, as explained in greater detail below. Exemplary materials suitable for use in the layer 90 include nonwovens, foams, woven materials, etc. In particular, the layer 90 may comprise, by way of illustration and not limitation, rayon, Lyocell and other cellulose-based materials, cotton, polyester, polypropylene and polypropylene blends (e.g., with other listed materials, such as a Lyocell/polypropylene blend), and hydrophilic forms of nonwovens such as SM (spunbond meltblown), SMS (spunbond meltblown spunbond), and SMMS (spunbond meltblown meltblown spunbond).

The layer 90 has first and second sides 92, 94 that may be parallel to the longitudinal axis 30 of the article 20. Preferably, but not necessarily, a first elastic member 96 may be attached to the layer 90 at the first side 92, while a second 98 elastic member may be attached to the layer 90 at the second side 94. The elastic members 96, 98 may extend along the entire length of the layer 90, or only a portion thereof. A fully or partially elasticized layer 90 may to tend to draw the sensation member 80 toward or against the skin of the wearer. Alternatively, the layer 90 may be formed to have a lesser length than another layer disposed relatively exteriorly, etc.

According to a first variation according to this embodiment, the sensation member 80 consists essentially of the hydrophilic layer 90. That is, it may be sufficient for the purpose of alerting the wearer to an insult of urine that the urine be maintained for a period of time in the layer 90, thereby providing a sensation to the wearer. However, according to other variations of this embodiment, an active component, such as coating or agent, may be applied at 100a, 100b to the layer 90, which may be referred to as a support layer, as illustrated in solid and dashed line.

Thus, according to a second variation, the sensation member 80 may also comprise an active component in the form of a hydrophilic coating, which may be applied at 100a as shown in solid line in Fig. 3b. The hydrophilic coating may be disposed in a face-to-face arrangement with the support layer 90. Moreover, as illustrated, the hydrophilic coating may be disposed on the surface of the layer 90 closer to the wearer's skin (i.e., body-facing surface).

It will be also recognized that the hydrophilic coating may include a diverse range of materials, including lotions, creams and the like. Exemplary hydrophilic coatings include surfactants, such as the NUWET silicone surfactant available from GE Silicones of Wilton, CT.

Further, according to a third variation, the sensation member 80 may also comprise an active component in the form of a temperature sensation agent (composition or structure), which may be applied at 100a. The temperature sensation agent may be disposed in face-to-face arrangement with the support layer 90. Moreover, as illustrated, the temperature sensation agent may be disposed on the surface of the layer 90 closer to the wearer's skin (i.e., body-facing surface). Further, the temperature sensation agent may be disposed on the support layer 90 in place of the hydrophilic coating, in conjunction with the hydrophilic coating, or combined (e.g., mixed) with the hydrophilic coating.

It will be recognized that the temperature sensation agent may include those materials that produce a temperature change (i.e., involve an endothermic or an exothermic reaction), as well as those that produce the sensation that a temperature change has occurred without actually producing a temperature change. For example, the temperature sensation agent may be a cooling agent. Further, the cooling agent may be the AQUACOOL dye manufactured by United Polymer Technology of Akron, Ohio. The AQUACOOL dye is a water-soluble dye that changes temperatures when brought into contact with water. Another example of cooling agent may be menthol or a menthol derivative, which chemicals are believed to provide the sensation of a temperature change, while not actually producing a temperature change. The COOLACT P and COOLACT 10 products manufactured by LIPO Chemicals of Paterson, New Jersey are examples of menthol derivative products which may be suitable. Other examples of temperature change agents (e.g., endothermic salts) that may be suitable temperature sensation agents may be found in U.S. Patent No. 6,642,427.

Moreover, according to a fourth variation, the sensation member 80 may comprise an active component in the form of a hydrophobic coating, which may be applied at 100b as shown in dashed line in Fig. 3b. According to this variation, the hydrophilic coating and/or temperature sensation agent may or may not be included (the temperature sensation agent being combinable with either the hydrophilic or hydrophobic agent, if present). Like the hydrophilic coating, the hydrophobic coating may be disposed in a face-to-face arrangement with the support layer 90. Moreover, as illustrated, the hydrophobic coating may be disposed on the surface of the layer 90 between the layer 90 and the topsheet 60, or the surface further from the wearer's skin (i.e., the opposite surface).

It will be also recognized that the coating 100b may include a diverse range of materials, including lotions, creams etc. Exemplary coatings may comprise hydrophobic coatings (HFC) and liquid-impermeable surface coatings (LISC). In particular, the coating may be made in accordance with the disclosure of U.S. Published Application No. 2005/0177123. Alternatively, the coatings may be acrylic polymer (e.g., acrylamide, ethyl alcohol, n-butyl alcohol, methylmethacrylate, acrylamide, acrylonitrile, or combinations thereof) emulsions manufactured and sold, for example, under the ROHATOL tradename by Lanxess Corp. of Pittsburg, Pa, the RH-MW1845K tradename by Rohm & Haas of Philadelphia, Pennsylvania, or the FA1, FA2, or FA3 tradenames by PolymerLatex International GmbH of Marl, Germany.

In fact, the hydrophilic and hydrophobic coatings and temperature sensation agents described above may be used with other sensation members, as will be discussed below. Moreover, the coatings and agents may be useful in conjunction with the structures described in U.S. Patent No. 6,627,786, among others.

The spacing of the first and second sides 92, 94 of the layer 90 and the width of the coating, if present, may be determined to allow enough liquid to bypass the sensation member 80 to the core 64 so as to prevent flooding. Flooding may result in leakage of the article 20 during urination, which is undesirable in the article 20 when it is a diaper or training pant, for example. Consequently, it will be recognized that the dimensions of the layer 90 and coating may be determined to prevent flooding while at the same time wicking sufficient liquid to create a sensation of wetness for the user.

During insults of urine, the layer 90 allows urine to penetrate in the z-direction and also provides a medium for the flow of urine in the x-y plane via wicking. The layer 90 and/or the coating may enhance the movement of the passage of the urine in the x-y plane, thereby expanding the wetted area of the sensation member, which preferably is held in contact with the wearer's skin. The wicking in the x-y plane causes the urine to spread out and effectively wet a large area before being absorbed into the absorbent assembly, thereby maximizing the wetness signal experienced by the wearer.

In fact, the sensation member 80 may have a high initial wetness that dries out after, for example, approximately 10 minutes. That is, while the initial wetness may vary whether the layer 90 is used alone, or in combination with a hydrophilic or hydrophobic coating, the preferred response would be for the initial wetness to be sufficient to cause the wearer to recognize the condition, and the wetness over time to be limited so as not to create, for example; skin health issues because of too much wetness being present near the skin over a prolonged period of time.

The disposable absorbent article 20 may have visible highlighting, indicated at 110 in Figure 3a and illustrated as an exemplary pattern of wavy lines and circles, in the interior of the article associated with the sensation member or members 80 to indicate the presence of the sensation member or members 80 and thereby facilitate an opportunity for the urinary toilet training of the wearer of the article. Such visible highlighting is described in U.S. Published Application No. 2005/0096612. Although a sensation member lacking this visible highlighting is fully functional in terms of providing a noticeable wetness and/or temperature signal to the wearer, the caregiver might overlook or forget the possibility of capitalizing on each opportunity for urinary toilet training if the body-facing portion of the absorbent article presents a generally uniform appearance, such as in absorbent articles that present a generally uniform white appearance on their body-facing surfaces.

Furthermore, once the caregiver decides to mention urinary toilet training to the wearer, the visible highlighting can serve to draw the wearer's interest or can be pointed out by the caregiver and incorporated into an explanation of the upcoming opportunity. Thus, the visible highlighting can provide a topic for conversation between the caregiver and the wearer on the subject of urinary toilet training and can likewise provide a nameable object for reference by the wearer, greatly simplifying the mental task required of the wearer who desires to communicate his or her need to go to the toilet or to communicate his or her improving recognition of the wetness signal provided by the sensation member.

Even a simple solid coloring form of visible highlighting can serve to facilitate an opportunity for urinary toilet training, especially when used with wearers possessing some recognition of colors or colored forms. In addition, visible highlighting in the form of a color or colors may facilitate the teaching of recognition of colors and differences between colors, and the associated learning may enhance the urinary toilet training process in turn.

Because the sensation member is located in what may be generally termed the laterally central region of the absorbent article, visibly highlighting the sensation member may provide additional benefits related to the learning achieved by the wearer. For example, a visibly highlighted sensation member may provide a line of reference for the visual separation of the two leg openings, including their differentiation into right and left leg openings for the respective feet to be inserted into the corresponding leg openings. Similarly, a longitudinally oriented visible highlighting may serve as a visual reference for the front to back direction, both for orienting the article prior to applying it, if done by the caregiver, or prior to donning it, if done by the wearer. This longitudinally oriented visual reference may also aid in the teaching of such skills as wiping one's self clean after using the toilet by using a longitudinal motion. The concept of something being central or "in the middle" may be taught and learned by visual reference to the visible highlighting and this concept may then be applied to related subjects, such as the anatomical location of the source of urine and the corresponding proper position in which to sit on the toilet. Thus, in the above and similar ways, the wearer can be made more aware of his or her own body, which may tend to enhance and facilitate the urinary toilet training experience.

In addition, the visible highlighting can serve to enhance the self-esteem of the wearer through a reminder that he or she is mature enough to be engaged in urinary toilet training. This effect can be compounded when the wearer succeeds in recognizing the need to go to the toilet and then sees the dry condition of the visibly highlighted sensation member inside the article after pulling it down.

The visible highlighting may be provided by means of printing onto a surface of the sensation member or one of its layers. For example, solid coloring or a graphic may be printed onto a surface of the coating underlying the water-permeable layer. As another example, an adhesive or a gel may be printed onto a surface of either of the two layers. Such an adhesive or gel may be colored differently from the surrounding area. Alternatively, the adhesive or gel may be uncolored or may have the same color as the surrounding area, but may still provide visible highlighting by forming a distinctive raised area or pattern and/or by surrounding a distinctive recessed area or pattern.

The visible highlighting may also be provided by forming one or more layers of the sensation member of a colored material, for example, a fibrous layer containing colored fibers, a monolithic layer containing a dispersed or imbedded colorant, a layer of an unbleached material that is colored in its virgin state, and so on.

In some embodiments, the visible highlighting may be provided by impressing or embossing the sensation member or one of it layers. The impressed, embossed, or bonded portions of the sensation member may provide a tactile sensation in addition to visibly highlighting the presence and location of the sensation member. For instance, a raised area or a recessed area or the combination of raised and recessed areas adjacent to each other may be felt by the hand and, in some embodiments, may be felt by the wearer while wearing the article. Similarly, the raised area or pattern formed by a printed adhesive or gel, as mentioned above, may provide such a tactile sensation. Just as with the visible highlighting alone, the combination of visible highlighting and this tactile sensation can serve to draw the wearer's interest or can be pointed out by the caregiver and incorporated into an explanation of the upcoming opportunity for urinary toilet training.

In addition, the visible highlighting may be provided by incorporating distinctive fibers or filaments in one or both layers of the sensation member or by distinctively orienting fibers or filaments in one of these layers. For example, a fiber or a filament of a distinctive color may be incorporated into the coating to visibly highlight its presence and its location in the article. Similarly, a distinctively thicker fiber or filament may be embedded in one of the two layers and thereby form a distinctive raised area or pattern.

If the portions of the structure of the absorbent article surrounding the sensation member are of one color, the visible highlighting can be provided by the use of another color, by the use of contrast, by the use of a different pattern in the same or a similar color, or by any other method that visibly differentiates the sensation member from the surrounding structural elements.

In some embodiments, the visible highlighting may include more than one color, more than one difference in contrast, more than one pattern, more than one graphic, more than one area of solid coloring, and so on, such that all portions of this description referring to the singular of a form of visible highlighting are meant to include the plural, and vice versa.

The visible highlighting may include open or closed geometric figures, a two dimensional representation of a three dimensional object, a representation of a commonly named or nameable shape or object, a representation of a recognizable object used in play, and/or a representation of a character that may be known to the wearer, such as a teddy bear, a character appearing on a television show for children, a character appearing in a game or a storybook for children, etc. In embodiments in which the visible highlighting includes a variety of figures, objects, and/or characters, the various elements of the visible highlighting may be interactively interrelated, related by subject matter, and/or related by a common story line. Conversely, the various elements may be interactively unrelated, unrelated by subject matter, and/or not related by a common story line.

When solid coloring is used, it may partially or completely fill the area bounded by a graphic outline, appear as shading inside or outside such a graphic outline, itself form a "filled-in" graphic, or simply uninterruptedly occupy an area, e.g., occupy the entire width of a layer of the sensation member over all or a portion of the corresponding length.

In some embodiments, the visible highlighting may become more or less visible when the sensation member is wetted. In addition, the visible highlighting may change color when the sensation member is wetted. Any of these effects may be created by the use of inks or dyes or other agents that undergo chemical reactions or are dispersed or concentrated when wetted by urine. In general, any of the wetness indicating compositions commonly used in externally visible wetness indicators, such as so-called "appearing" or "disappearing" wetness indicators that may become more or less visible when wetted and in wetness indicators that may change color when wetted, may be used for these versions of visible highlighting.

It is important to note that rather than being structurally disposed in such a way as to provide a wetness indication that is visible from the outside of the absorbent article, according to at least one embodiment, any wetness indicating compositions used for the visible highlighting of the sensation member should be visible from the body-facing surface of the absorbent article. This different disposition enables the caregiver to apply different techniques to the task of urinary toilet training when using an absorbent article of the present disclosure, as compared to using an absorbent article having only a wetness indicator visible from the outside of the article. For example, while the change in an exterior wetness indicator is visible for all to see, any change in the visible highlighting of an interior sensation member remains "private" until either the caregiver or the wearer peers into the absorbent article or it is removed. Therefore, whether or not any wetting of the absorbent article has occurred can, itself, become the focus of a playful activity resembling a game, with the "secret" being revealed only when the caregiver and the wearer agree to conclude the game. If the wearer notices a sensation of wetness or merely desires to check the condition of the "private" indication, he or she can simply look inside the absorbent article. If the appearance of the visible highlighting has changed, the wearer can then choose to bring this to the attention of the caregiver in the context of asking to go to the bathroom. In addition, because the visible highlighting serves as a "private" indication, the wearer might be able to detect a change in its appearance before the appearance of any externally visible wetness indicator changes and thereby be the first person to mention the subject of going to the toilet. Furthermore, the provision of both visual and tactile sensations to the wearer may serve to reinforce the tactile sensation of wetness and thereby enhance the training effect of the sensation member. An absorbent article in which the wetting is indicated by both a sensation and a visible change in the appearance of the visible highlighting may thus facilitate faster learning on the part of the wearer.

Although the appearance of the visible highlighting remains "private" until either the caregiver or the wearer peers into the absorbent article or it is removed, the visible highlighting may be associatively correlated in visible form with marking that is located elsewhere in or on the absorbent article and is visible from the outside of the absorbent article. This externally visible marking may be permanent or may change in appearance while the absorbent article is being worn. For example, the externally visible marking may be an externally visible wetness indicator. By giving the visible highlighting of the sensation member a visible form that is similar to the visible form of an externally visible marking, an opportunity for urinary toilet training may be enhanced. For instance, the caregiver can point out the similarity between the externally visible marking and the "private" visible highlighting of the sensation member and ask the wearer to remember the hidden visible highlighting every time he or she notices the externally visible marking.

For example, the article 20 may comprise an internal graphic 110, a first external graphic, and a second external graphic. The internal graphic may be permanent, while the external graphics may be "appearing" or "disappearing." The first external graphics may include a character iniage resembling a boy and a text graphic including words forming a message, such as "Remember to go to the potty!" While the graphics may include text, the primary form of communication may be symbols, icons, or other markings other than words, so that a pre-literate child may comprehend and follow the instructions or other information indicated by the graphics, although it is not necessary for the images to be understood at this level. The second external graphics may include an image that may be associatively correlated to the permanent graphic, such as a dog or stars.

Variations regarding the internal/external graphics are possible. For example, a permanent external image may be combined with the first and second external graphics, or only one external graphic may be included. Furthermore, character images other than a boy may be provided, such as a girl, an animal (which may be anthropomorphic), a cartoon character, and the like. Still further, additional or alternative text may be provided. Additionally exemplary graphics, graphics characteristics and/or arrangements (e.g., timings, themes, scenes, storylines, etc.), the materials that are suitable for forming the graphics, and the arrangement and/or joining of these materials to the article 20 are described in co-pending and commonly assigned U.S. Patent Application No. 11/098,362, filed in the name of Roe et al. on April 4, 2005.

Even in embodiments in which the appearance of the visible highlighting is not affected by its being wetted, the associative correlation of the respective visible forms of an externally visible marking and the visible highlighting may serve to facilitate an opportunity for urinary toilet training. For example, if both the externally visible marking and the visible highlighting have the visible form of similar graphics, the externally visible marking can serve to draw the wearer's interest or can be pointed out by the caregiver and incorporated into an explanation of the ongoing opportunity for urinary toilet training.

Such associative correlation of the respective visible forms of an externally visible marking and the visible highlighting can be achieved without the respective visible forms being similar, so long as the respective visible forms are mutually related in a recognizable way. For example, the visible forms may be related in subject matter and/or may be related by a common story line and/or be interactively interrelated. Even an associative correlation of a simple solid coloring form of an externally visible marking with a similar solid coloring form of visible highlighting can serve to facilitate an opportunity for urinary toilet training, especially when used with wearers possessing some recognition of colors or colored forms.

Alternatively, the visible highlighting may be associatively uncorrelated with any externally visible marking. The lack of associative correlation may be complete or may be specific, e.g., the respective visible forms of the visible highlighting and the externally visible marking may be unrelated in subject matter, not related by a common story line, and/or interactively unrelated, while still being associatively correlated in another way.

The visible form of the visible highlighting of the sensation member need not be associatively correlated with the concept of urinary toilet training. However, in some embodiments, the visible form of the visible highlighting may be associatively correlated with the concept of urinary toilet training by, for example, providing a visual reference to the liquid-related nature of urinary toilet training, such as wetness, dryness, protection from wetness, the flow of a liquid, water, et cetera, and thus may serve to facilitate an opportunity for urinary toilet training.

The visible highlighting may emphasize dryness by depicting the sun, fair weather clouds, a sunny day, etc., while wetness may be referenced by a depiction of a water puddle, a cloud with falling rain, etc. A visual reference to protection from wetness may be provided by a depiction of an umbrella, a raincoat, a rain hat, galoshes, a submarine, or some other object that may be associated by the wearer with the concept of staying dry in a wet environment.

In any of these visible forms of visible highlighting that are associatively correlated with the concept of urinary toilet training, a human form and/or a recognizable character may be depicted in the visible highlighting. For example, a child may be shown in conjunction with inanimate objects, a child may be shown sitting on a potty chair, and/or a character from a children's storybook or a children's television program may be shown in similar poses, etc.

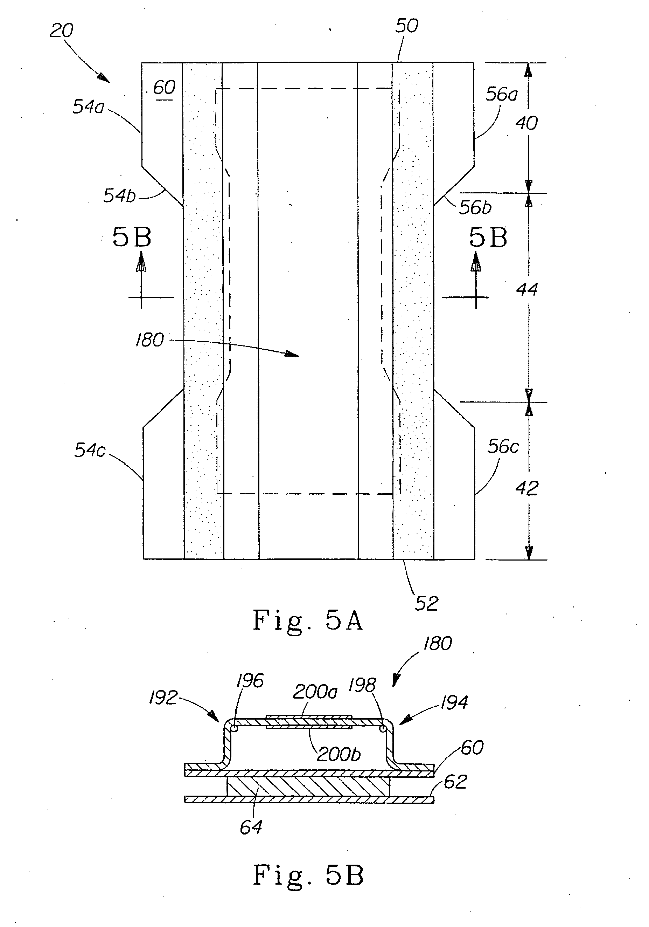

Turning next to Figs. 5a and 5b, a second embodiment of a sensation member 180 is illustrated therein. Similar to the sensation member 80, the sensation member 180 has first and second sides 192, 194 that are arranged parallel to the longitudinal axis 30 of the article 20. Moreover, elastic members 196, 198 may be attached to the sensation member 180 at the sides 192, 194 so as to elasticize the sensation member 180, which may assist in bringing the sensation member 180 in to close contact with the skin of the wearer. Further, the sensation member may include an active component; such as (i) a coating, which may be a hydrophilic coating disposed on a body-facing surface or a hydrophobic coating disposed on an opposite surface, (ii) a temperature sensation agent, which may be disposed on either surface and in substitution for, in conjunction with, or combined with the coating, and/or (iii) a layer of hydrophilic material, such as was described relative to the layer 90 above and which also may be disposed on a body-facing surface.

The sensation member 180 differs from the sensation member 80 in that the structure corresponding to the support layer 90 is formed from a section of the topsheet 60 spaced from the core 64. That is, a section of the topsheet 60 is folded to define support layer structure of the member 180, and, in particular, is folded along the sides 192, 194. The elastic members 196, 198 are then disposed beneath the topsheet 60 in the space between the topsheet 60 and the core 64. In this fashion, the sensation member 180 may be integrated to a greater degree to the remainder of the article 20 than the member 80, thereby reducing the likelihood that the sensation member 180 will become detached from the remainder of the article 20.

The sensation member 180 may include other features in common with the sensation member 80. For example, the coatings and agents disposed at 200a, 200b may include those exemplary coatings and agents listed above. Moreover, while not illustrated, a visible graphic 110 may be include on a surface of the sensation member 180, providing one or more of the advantages discussed above.

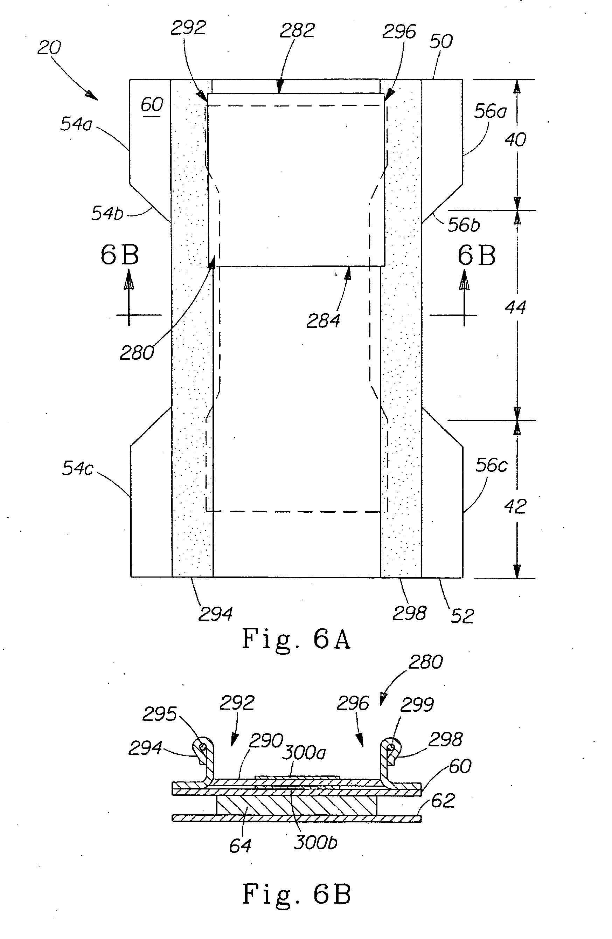

Turning then to Figs. 6a and 6b, a third embodiment of the sensation member 280 is illustrated, with the barrier leg cuffs folded back slightly in Fig. 6a to expose the sensation member 280. The sensation member 280 has a first laterally extending end 282 and a second longitudinally opposing and laterally extending end 284. As will be recognized, the distance between the ends 282, 284 is shorter than the distance between the ends 50, 52, or even the distance between end 50 and the crotch region 44. According to the embodiment, the position of the ends 282, 284 relative to the ends 50, 52 and the spacing between the ends 282, 284 is such that the likelihood that the sensation member 280 will be wetted with urine is enhanced.

The sensation member 280 includes a layer 290. The layer 290 extends between the ends 282, 284. Additionally, a first longitudinal edge 292 of the layer 290 is attached to a first barrier leg cuff 294 attached to the topsheet 60, while a laterally opposed, longitudinal edge 296 is attached to a second, spaced barrier leg cuff 298, also attached to the topsheet 60. Moreover, each barrier leg cuff 294, 298 includes an elastic member 295, 299. In this fashion, it is not necessary to attach separate elastic members to the support layer 290, but the elastic members 295, 299 of the barrier leg cuffs 294, 298 instead may urge the sensation member 280 into contact with the skin of the wearer.

In fact, it is believed that the attachment of the sensation member 280 to the barrier leg cuffs 294, 298 may permit greater control over the spacing of the sensation member 280 relative to the topsheet 60 (i.e., distance between member 280 and topsheet 60) than had heretofore been possible. That is, by attaching the sensation member 280 along its sides 292, 296, rather than at its ends 282, 284, the spacing of the member 280 relative to the topsheet 60 may be better controlled than in those embodiments wherein the member is attached at its ends, or potentially even in those embodiments where the member is integrated into the topsheet 60 and elastic members disposed internal to the topsheet 60 are used to define, at least in part, the sensation member. Additionally, by attaching the sensation member 280 to the leg cuffs 294, 298, the dimension of the sensation member 280 perpendicular to the longitudinal axis may be greater than, for example, the sensation members 80, 180 discussed above.

Similar to the embodiment shown in Figs. 3a and 3b, the sensation member 280 may consist essentially of the layer 290, or may comprise the layer 290 in combination with a coating or an agent, which coating or agent may be disposed in a face-to-face arrangement with the layer 290, which may be referred to as a support layer. The coating may be a hydrophilic coating disposed at 300a on the body-facing surface of the layer 290, or a hydrophobic coating disposed at 300b on the opposite surface. Additionally, a temperature sensation agent may be disposed at 300a on either surface and in substitution for, in conjunction with, or combined with the coating.

The sensation member 280 may include other features in common with the sensation member 80, 180. For example, the coatings and agents may include those exemplary coatings and agents listed above. Additionally, while not illustrated, a visible graphic 110 may be include on a surface of the sensation member 280, providing one or more of the advantages discussed above.

In addition to the features described above, the disposable absorbent article 20 may also include a variety of features known in the art, such as slit openings, outer leg cuffs, front and rear ear panels, waist cap features, elastics, and the like to provide desired fit, containment, and aesthetic characteristics. Such additional features are well known in the art and are described in U.S. Patent Nos. 3,860,003, 5,151,092, and 6,482,191 among others. Additionally, a transfer layer, which may also be referred to as an acquisition or distribution layer, may be disposed between the topsheet 60 and the core 64. Moreover, the elements discussed above may be modified from their illustrated forms.

## Claims

1. An absorbent article having a waist region and a crotch region, the article comprising:
a backsheet having a longitudinal axis;
a topsheet attached to the backsheet and having a body-facing surface;
an absorbent core disposed between the backsheet and the topsheet; **characterized in that** the article comprises
a sensation member with first and second joining regions spaced from each other along the longitudinal axis, the first joining region being attached to the topsheet at the waist region and the second joining region being attached to the topsheet at the crotch region,
the sensation member consisting essentially of a layer of hydrophilic material; and
wherein the sensation member includes a support layer having a body-facing surface and an opposite surface facing the topsheet, and an active component on one of the body-facing surface or the opposite surface of the sensation member;
wherein the active component is on the body-facing surface and comprises a hydrophilic coating; or
wherein the active component is on the opposite surface and comprises a hydrophobic coating.

2. The absorbent article according to claim 1, wherein the sensation member has first and second sides, and comprising first and second elastic members disposed along the first and second sides.

3. The absorbent article of claim 1 further comprising first and second spaced barrier leg cuffs attached to the topsheet parallel to the longitudinal axis;
a sensation members having first and second spaced sides, the first side being attached to the first barrier leg cuff and the second side being attached to the second barrier leg cuff with the sensation member spaced a distance from the body-facing surface of the topsheet.

4. The absorbent article according to claim 1, comprising a visible highlight associated with the sensation member.

5. The absorbent article according to claim 1, wherein the articles is a diaper, pant or refastenable pant.
<
>

6. The absorbent article according to claim 1, wherein the active component is on the body-facing surface and comprises a temperature sensation agent.

7. The absorbent article according to claim 1, wherein the sensation member has a third joining region spaced from the first and second joining regions along the sensation member longitudinal axis, the third joining region attached to the topsheet at the waist region opposite the first joining region.

## Patentansprüche

1. Absorptionsartikel mit einem Taillenbereich und einem Schrittbereich, wobei der Artikel Folgendes umfasst:
eine Unterschicht mit einer Längsachse;
eine mit der Unterschicht verbundene Oberschicht mit einer körperseitigen Oberfläche;
einen zwischen der Unterschicht und der Oberschicht angeordneten Absorptionskern; **dadurch gekennzeichnet, dass** der Artikel Folgendes umfasst:
ein Wahrnehmungselement mit einem ersten und einem zweiten Verbindungsbereich, die entlang der Längsachse voneinander beabstandet sind, wobei der erste Verbindungsbereich im Taillenbereich mit der Oberschicht verbunden ist und der zweite Verbindungsbereich im Schrittbereich mit der Oberschicht verbunden ist,
wobei das Wahrnehmungselement im Wesentlichen aus einer Schicht aus hydrophilem Material besteht; und wobei das Wahrnehmungselement eine Stützschicht mit einer körperseitigen Oberfläche und einer gegenüberliegenden, der Oberschicht zugewandten Oberfläche und eine Wirkkomponente an entweder der körperseitigen Oberfläche oder der gegenüberliegenden Oberfläche des Wahrnehmungselements einschließt; wobei sich die Wirkkomponente an der körperseitigen Oberfläche befindet und eine hydrophile Beschichtung umfasst oder wobei sich die Wirkkomponente an der gegenüberliegenden Oberfläche befindet und eine hydrophobe Beschichtung umfasst.

2. Absorptionsartikel nach Anspruch 1, wobei das Wahrnehmungselement eine erste und eine zweite Seite besitzt und ein erstes und ein zweites elastisches Element umfasst, die entlang der ersten und der zweiten Seite angeordnet sind.

3. Absorptionsartikel nach Anspruch 1, ferner umfassend ein erstes und ein zweites voneinander beabstandetes Sperrbeinbündchen, die parallel zu der Längsachse an der Oberschicht befestigt sind;
ein Wahrnehmungselement mit einer ersten und einer zweiten voneinander beabstandeten Seite, wobei die erste Seite an dem ersten Sperrbeinbündchen befestigt ist und die zweite Seite an dem zweiten Sperrbeinbündchen befestigt ist, wobei das Wahrnehmungselement von der körperseitigen Oberfläche der Oberschicht beabstandet ist.

4. Absorptionsartikel nach Anspruch 1, umfassend eine zu dem Wahrnehmungselement gehörende optische Hervorhebung.

5. Absorptionsartikel nach Anspruch 1, wobei der Artikel eine Windel, eine Hose oder eine wiederverschließbare Hose ist.

6. Absorptionsartikel nach Anspruch 1, wobei die Wirkkomponente auf der körperseitigen Oberfläche liegt und ein Temperaturwahrnehmungsmittel umfasst.

7. Absorptionsartikel nach Anspruch 1, wobei das Wahrnehmungselement einen dritten Verbindungsbereich aufweist, der vom ersten und zweiten Verbindungsbereich entlang der Längsachse des Wahrnehmungselements beabstandet ist, wobei der dritte Verbindungsbereich im Taillenbereich gegenüber dem ersten Verbindungsbereich an der Oberschicht befestigt ist.

## Revendications

1. Article absorbant ayant une région de ceinture et une région d'entrejambe, l'article comprenant :
une feuille de fond ayant un axe longitudinal ;
une feuille de dessus fixée à la feuille de fond et ayant une surface faisant face au corps ;
une âme absorbante disposée entre la feuille de fond et la feuille de dessus ;
**caractérisé en ce que** l'article comprend
un élément de sensation avec des première et deuxième régions de raccordement espacées l'une de l'autre le long de l'axe longitudinal, la première région de raccordement étant fixée à la feuille de dessus au niveau de la région de ceinture et la deuxième région de raccordement étant fixée à la feuille de dessus au niveau de la région d'entrejambe,
l'élément de sensation étant constitué sensiblement d'une couche de matériau hydrophile ; et dans lequel l'élément de sensation inclut une couche de support ayant une surface faisant face au corps et une surface opposée faisant face à la feuille de dessus, et un composant actif sur une parmi la surface faisant face au corps ou la surface opposée de l'élément de sensation ; dans lequel le composant actif est sur la surface faisant face au corps et comprend un revêtement hydrophobe ; ou dans lequel le composant actif est sur la surface opposée et comprend un revêtement hydrophobe.

2. Article absorbant selon la revendication 1, dans lequel l'élément de sensation a des premier et deuxième côtés, et comprenant des premier et deuxième éléments élastiques disposés le long des premier et deuxième côtés.

3. Article absorbant selon la revendication 1, comprenant en outre un premier et un deuxième revers de jambe de barrière espacés fixés à la feuille de dessus parallèles à l'axe longitudinal ;
un élément de sensation ayant des premier et deuxième côtés espacés, le premier côté étant fixé au premier revers de jambe de barrière et le deuxième côté étant fixé au deuxième revers de jambe de barrière avec l'élément de sensation espacé d'une distance par rapport à la surface faisant face au corps de la feuille de dessus.

4. Article absorbant selon la revendication 1, comprenant une accentuation visible associée à l'élément de sensation.

5. Article absorbant selon la revendication 1, où l'article est une couche, une culotte ou une culotte refermable.

6. Article absorbant selon la revendication 1, dans lequel le composant actif est sur la surface faisant face au corps et comprend un agent de sensation de température.

7. Article absorbant selon la revendication 1, dans lequel l'élément de sensation a une troisième région de raccordement espacée des première et deuxième régions de raccordement le long de l'axe longitudinal de l'élément de sensation, la troisième région de raccordement fixée à la feuille de dessus au niveau de la région de ceinture opposée à la première région de raccordement.
